# EUROPÄISCHE PATENTSCHRIFT

(11) **EP 2 044 014 B1**
(45) Veröffentlichungstag und Bekanntmachung des Hinweises auf die Patenterteilung: **18.08.2010**
(21) Anmeldenummer: 07786751.3
(22) Anmeldetag: 18.06.2007
(51) Int. Cl.: C07C 407/00, C07C 409/38

(54) **VERFAHREN ZUR HERSTELLUNG VON ACYLPEROXIDEN**
METHOD FOR PRODUCING ACYL PEROXIDES
PROCEDE DE PRODUCTION DE PEROXIDES D'ACYLE

(30) Priorität: 12.07.2006 DE 102006032166
(43) Veröffentlichungstag der Anmeldung: 08.04.2009
(73) Patentinhaber: United Initiators GmbH & Co. KG, 82049 Pullach (DE)
(72) Erfinder: APPEL, Hans, 82377 Penzberg (DE); MEICHELBÖCK, Wilfried, 82152 Krailling (DE); WEINMAIER, Josef, Helmut, 84453 Mühldorf (DE); ZELLNER, Helmut, 82278 Hörbach (DE)
(74) Vertreter: Dey, Michael
(86) Internationale Anmeldenummer: PCT/EP2007/056013
(87) Internationale Veröffentlichungsnummer: WO 2008/006668

(56) Entgegenhaltungen:
- EP-A2- 0 478 214
- DE-A1- 1 950 536
- US-A- 2 567 615
- US-A- 3 849 468
- US-A- 5 012 010
- US-B1- 6 224 845
- MILAS, N.A. ET AL.: "Studies in organic peroxides. IX. t-Butyl peresters" J. AM. CHEM. SOC., Bd. 68, 1946, Seiten 642-643, XP002453451
- BLOMQUIST, A.T. ET AL.: "The kinetics of the thermal decomposition of peresters. III. The effect of p-substituents on the unimolecular decomposition of t-butyl perbenzoates" J. AM. CHEM. SOC., Bd. 73, 1951, Seiten 5546-5550, XP002453452

## Beschreibung

Die Erfindung richtet sich auf ein Verfahren zur Herstellung von Acylperoxiden aus organischen Hydroperoxiden, bei dem nicht umgesetztes Hydroperoxid zurückgewonnen und in die Reaktion zurückgeführt wird.

Acylperoxide werden üblicherweise durch Umsetzung einer Acylverbindung aus der Reihe der Carbonsäurechloride, Carbonsäureanhydride und Chlorformiate mit einem organischen Hydroperoxid hergestellt. Die Umsetzung erfolgt unter Zusatz einer wässrigen Lösung einer Base, um die bei der Umsetzung der Acylverbindung mit dem Hydroperoxid freiwerdende Säure zu binden. Die Umsetzung erfolgt dabei in einer zweiphasigen Reaktionsmischung und verläuft exotherm.

Für die spätere Anwendung müssen die Acylperoxide möglichst frei von der eingesetzten Acylverbindung sein. Deshalb wird üblicherweise das organische Hydroperoxid in stöchiometrischem Überschuss zur Acylverbindung eingesetzt, um einen möglichst vollständigen Umsatz an Acylverbindung zu erreichen. Bei der Umsetzung bleibt dann unumgesetztes organisches Hydroperoxid zurück.

US 2,567,615 offenbart die Herstellung von Peroxyestern durch Umsetzung eines Carbonsäurechlorids oder Carbonsäureanhydrids mit einem tertiären Alkylhydroperoxid in Gegenwart eines Alkalimetallhydroxids. Wie aus den Beispielen 8, 9 und 10 hervorgeht, bei denen das Alkylhydroperoxid im Überschuss verwendet wird, wird bei Waschen der bei der Umsetzung erhaltenen organischen Phase mit 10 Gew.-% Natriumcarbonat und mit Wasser ein Produkt erhalten, das neben dem Peroxyester noch nicht umgesetztes Alkylhydroperoxid enthält.

US 3,138,627 offenbart die analoge Herstellung von tert-Butylperoxyestern von niederen aliphatischen Carbonsäuren unter Verwendung eines Lösungsmittels. Das Dokument gibt dem Fachmann die Lehre, den Peroxyester möglichst rasch von der alkalischen Mischung abzutrennen, um eine Verseifung durch die basische Lösung zu vermeiden.

US 4,075,236 offenbart ein kontinuierliches Verfahren zur Herstellung von Peroxyestern, bei dem ein Carbonsäurechlorid mit einem Hydroperoxid und einem Alkalimetallhydroxid umgesetzt werden, wobei eine zweiphasige Reaktionsmischung erhalten wird. Bei der Umsetzung wird das Hydroperoxid in einem Überschuss von 0 bis 50 % eingesetzt. Der pH-Wert der wässrigen Phase liegt dabei im Bereich von 10 bis 14. Nach der Umsetzung wird die wässrige Phase abgetrennt und die organische Phase in mehreren Mixer-Settler-Stufen gewaschen. Die wässrigen Phasen werden alle verworfen. Eine Rückgewinnung von unumgesetztem Hydroperoxid wird nicht offenbart.

US 3,849,468 offenbart ein kontinuierliches Verfahren zur Herstellung von Acylperoxiden, bei dem in einer zweiphasigen Reaktionsmischung ein Carbonsäurechlorid mit Wasserstoffperoxid oder einem tertiären Hydroperoxid in Gegenwart eines Lösungsmittels umgesetzt werden, wobei Natriumhydroxid zugesetzt,wird, um einen pH-Wert im Bereich von 3 bis 10 einzustellen. Das tertiäre Hydroperoxid wird dabei in einem molaren Überschuss von 10 bis 20 % eingesetzt. Bei dem Verfahren erfolgt die Umsetzung in einem Schlaufenreaktor, dem bei jedem Umlauf der Reaktionsmischung die organische Phase und ein Teil der wässrigen Phase entnommen und einer Waschkolonne zugeführt wird, in der die organische Phase im Gegenstrom mit Wasser gewaschen wird. Die in der Waschkolonne anfallende wässrige Phase wird verworfen. Eine Rückgewinnung von unumgesetztem Hydroperoxid wird nicht offenbart.

Es besteht deshalb Bedarf für ein Verfahren zur Herstellung von Acylperoxiden aus organischen Hydroperoxiden, bei dem unumgesetztes Hydroperoxid zurückgewonnen und in die Reaktion zurückgeführt werden kann und mit dem ein Acylperoxid erhalten wird, das einen geringen Gehalt an unumgesetztem Hydroperoxid aufweist.

Gegenstand der Erfindung ist ein Verfahren zur Herstellung von Acylperoxiden durch Umsetzung einer Acylverbindung aus der Reihe der Carbonsäurechloride, Carbonsäureanhydride und Chlorformiate mit einem organischen Hydroperoxid, das folgende Schritte umfasst:
a) Umsetzung der Acylverbindung, des organischen Hydroperoxids und einer wässrigen Lösung einer Base unter Erhalt einer zweiphasigen Reaktionsmischung,
b) Einstellung des pH-Werts der wässrigen Phase der in Schritt a) erhaltenen Reaktionsmischung auf einen Wert im Bereich von 6 bis 13, vorzugsweise 11 bis 12,5,
c) Trennung der in Schritt b) erhaltenen zweiphasigen Mischung in eine wässrige und eine organische Phase,
d) Extraktion der in Schritt c) erhaltenen organischen Phase mit einer wässrigen Lösung einer Base und
e) Rückführung des in Schritt d) erhaltenen wässrigen Extrakts in Schritt a).

Das erfindungsgemäße Verfahren umfasst in Schritt a) die Umsetzung einer Acylverbindung aus der Reihe der Carbonsäurechloride, Carbonsäureanhydride und Chlorformiate mit einem organischen Hydroperoxid unter Zusatz einer wässrigen Lösung einer Base. Als Base können wasserlöslich Metallhydroxide, wasserlösliche quartäre Ammoniumhydroxide oder wasserlösliche tertiäre Amine verwendet werden. Bevorzugt wird als Base ein Alkalimetallhydroxid oder ein Erdalkalimetallhydroxid verwendet, besonders bevorzugt Natriumhydroxid oder Kaliumhydroxid. Bei der Umsetzung wird eine zweiphasige Reaktionsmischung erhalten, die eine organische Phase und eine wässrige Phase umfasst. Die organische Phase enthält das bei der Umsetzung gebildete Acylperoxid. Die wässrige Phase enthält das bei der Umsetzung der Acylverbindung mit dem Hydroperoxid gebildete Chloridsalz, bzw. Carboxylatsalz. Zusätzlich kann in der wässrigen Phase auch Carboxylatsalz enthalten sein, das durch Reaktion der Acylverbindung mit der Base gebildet wurde. Nicht umgesetztes organisches Hydroperoxid kann sowohl in der organischen Phase vorliegen, als auch in deprotonierter Form als Hydroperoxid-Salz in der wässrigen Phase vorliegen, wobei die Verteilung des Hydroperoxids zwischen organischer Phase und wässriger Phase vom pH-Wert der wässrigen Phase und dem Verteilungskoeffizient des organischen Hydroperoxids zwischen der wässrigen Phase und der organischen Phase abhängt.

Im nachfolgenden Schritt b) wird in der zweiphasigen Reaktionsmischung, die in Schritt a) erhalten wird, der pH-Wert der wässrigen Phase auf einen Wert im Bereich von 6 bis 13, vorzugsweise 11 bis 12,5, eingestellt. Der pH-Wert wird dabei vorzugsweise so niedrig gewählt, dass mehr als 50 %, besonders bevorzugt mehr als 80 %, des unumgesetzten organischen Hydroperoxids in der organischen Phase vorliegt und nur ein geringer Teil in deprotonierter Form als Hydroperoxid-Salz in der wässrigen Phase vorliegt. Durch die Wahl eines ausreichend niedrigen pH-Werts wird der Anteil an organischen Hydroperoxid, der in Schritt c) mit der wässrigen Phase abgetrennt wird, gering gehalten und so gewährleistet, dass der größte Teil des nicht umgesetzten Hydroperoxids in Schritt e) in die Reaktion zurückgeführt werden kann. Andererseits wird der pH-Wert vorzugsweise so hoch gewählt, dass in der Reaktionsmischung vorliegende Carbonsäure zu mehr als 80 %, besonders bevorzugt mehr als 90 %, in Form eines Carboxylatsalzes in der wässrigen Phase vorliegt und nur ein geringer Teil in Form der freien Carbonsäure in der organischen Phase vorliegt. Durch die Wahl eines ausreichend hohen pH-Werts wird verhindert, dass in Schritt d) unnötig Base durch die Deprotonierung von Carbonsäure verbraucht wird und in Schritt e) Carbonsäure in Form eines Carboxylatsalzes in die Reaktion zurückgeführt und dort akkumuliert wird.

Falls die in Schritt a) erhaltene Reaktionsmischung bereits eine wässrige Phase mit einem pH-Wert im erfindungsgemäßen Bereich aufweist und das organische Hydroperoxid, wie gewünscht, überwiegend in der organischen Phase vorliegt, ist in Schritt b) kein Zusatz von Base oder Säure erforderlich. In der Regel wird in Schritt b) ein Zusatz von Säure erforderlich sein, um den gewünschten pH-Wert und die gewünschte Verteilung von organischem Hydroperoxid zwischen wässriger Phase und organischer Phase zu erzielen. Vorzugsweise wird dann in Schritt b) eine Mineralsäure, besonders bevorzugt Schwefelsäure oder Salzsäure, zugesetzt, um den pH-Wert einzustellen. Bei Verwendung eines Säureanhydrids als Acylverbindung kann jedoch auch ein Zusatz einer Base erforderlich werden, um den gewünschten pH-Wert zu erzielen.

Anschließend wird dann in Schritt c) die in Schritt b) erhaltene zweiphasige Mischung in eine wässrige und eine organische Phase getrennt. Durch die in Schritt b) vorgenommene Einstellung des pH-Werts enthält die in Schritt c) abgetrennte wässrige Phase nur geringe Mengen an organischem Hydroperoxid und kann deshalb in der Regel ohne weitere Vorbehandlung einer biologischen Abwasserbehandlung zugeführt werden. Die wässrige Phase enthält dagegen den größten Teil des bei der Reaktion gebildeten Carboxylatsalzes, das in Schritt c) vom Acylperoxid abgetrennt wird.

Danach wird in Schritt d) die in Schritt c) erhaltene organische Phase mit einer wässrigen Lösung einer Base extrahiert. Die Konzentration der Base wird vorzugsweise so gewählt, dass bei der Extraktion der größte Teil, besonders bevorzugt mehr als 95 %, des in der organischen Phase enthaltenen organischen Hydroperoxids in Form des Hydroperoxid-Salzes in die wässrige Phase extrahiert und so vom Acylperoxid abgetrennt wird, das in der organischen Phase bleibt. Die Menge der zur Extraktion verwendeten wässrigen Lösung einer Base wird vorzugsweise so gewählt, dass die darin enthaltene Menge an Base nicht größer ist, als die in Schritt a) für die Umsetzung erforderliche Menge an Base.

Anschließend wird in Schritt e) der in Schritt d) erhaltene wässrige Extrakt mit dem darin in Form des Hydroperoxid-Salzes enthaltenen organischen Hydroperoxid in den Schritt a) zurückgeführt. Durch die Rückführung des nicht umgesetzten organischen Hydroperoxids kann auch bei im Überschuss eingesetztem organischen Hydroperoxid ein nahezu vollständiger Umsatz des Hydroperoxids zum Acylperoxid erreicht werden.

Das erfindungsgemäße Verfahren kann in den einzelnen Schritten sowohl kontinuierlich als auch diskontinuierlich durchgeführt werden, wobei auch kontinuierliche Schritte mit diskontinuierlichen Schritten kombiniert werden können.

In Schritt a) erfolgt die Umsetzung von Acylverbindung und organischem Hydroperoxid vorzugsweise in einem oder mehreren durchmischten Reaktoren, in denen eine gute Dispergierung der zweiphasigen Reaktionsmischung unter Ausbildung einer hohen Phasengrenzfläche gewährleistet ist. Geeignete durchmischte Reaktoren sind beispielsweise Rührkesselreaktoren, Schlaufenreaktoren oder Rohrreaktoren mit turbulenter Strömung, wobei die Turbulenz gegebenenfalls durch Einbauten erzeugt werden kann. Vorzugsweise werden gekühlte Reaktoren verwendet, um die freiwerdende Reaktionswärme bei annähernd konstanter Temperatur abzuführen. Besonders bevorzugt wird die Umsetzung in Schritt a) kontinuierlich in einer Anordnung aus mehreren in Reihe geschalteten durchmischten Reaktoren durchgeführt.

Bei der Herstellung von Acylperoxiden, die unter den verwendeten Reaktionsbedingungen flüssig sind, wird die Umsetzung in Schritt a) vorzugsweise ohne Lösungsmittel durchgeführt.

Acylverbindungen und/oder organische Hydroperoxide, die unter den verwendeten Reaktionsbedingungen fest sind, werden in Schritt a) vorzugsweise in Form einer Lösung in einem Lösungsmittel eingesetzt. Als Lösungsmittel eignen sich dabei alle dem Fachmann bekannten Lösungsmittel, die unter den Reaktionsbedingungen weder mit der Acylverbindung, noch mit dem organischen Hydroperoxid oder der Base reagieren. Vorzugsweise werden Lösungsmittel eingesetzt, die bei der verwendeten Reaktionstemperatur eine Löslichkeit in Wasser von weniger als 1 g/l aufweisen. Geeignete Lösungsmittel sind zum Beispiel Toluol und Isododecan.

Bei der Herstellung von Acylperoxiden, die unter den verwendeten Reaktionsbedingungen fest sind, wird vorzugsweise ein Lösungsmittel für das Acylperoxid in einer Menge zugesetzt, die gewährleistet, dass in Schritt a) als organische Phase eine flüssige Lösung des Acylperoxids in dem Lösungsmittel erhalten wird. Vorzugsweise werden Lösungsmittel eingesetzt, die bei der verwendeten Reaktionstemperatur eine Löslichkeit in Wasser von weniger als 1 g/l aufweisen. Geeignete Lösungsmittel sind zum Beispiel Toluol und Isododecan.

Das Molverhältnis von organischem Hydroperoxid zu Acylverbindung liegt bei der Umsetzung in Schritt a) vorzugsweise im Bereich von 1,01 : 1 bis 2 : 1, besonders bevorzugt 1,05 : 1 bis 1,5 : 1. Dieses Molverhältnis schließt die Menge an organischem Hydroperoxid mit ein, die durch die Rückführung des wässrigen Extrakts in Schritt e) in die Umsetzung zurückgeführt wird. Der Überschuss an organischem Hydroperoxid gegenüber der Acylverbindung wird dabei vorzugsweise so gewählt, dass in Schritt a) ein Umsatz der Acylverbindung von mehr als 90 %, besonders bevorzugt mehr als 93 % erreicht wird.

Bei der Umsetzung eines Carbonsäurechlorids der Struktur R¹C(O)Cl oder eines Carbonsäureanhydrids der Struktur R¹C(O)OC(O)R¹ mit einem Hydroperoxid der Struktur R²OOH wird in Schritt a) ein Percarbonsäureester der Struktur R¹C(O)OOR² erhalten. Die Acylverbindung und das Hydroperoxid werden dabei vorzugsweise in einem Molverhältnis von 1 : 1,01 bis 1 : 2, besonders bevorzugt 1 : 1,01 bis 1 : 1,5 eingesetzt. Das Molverhältnis von Acylverbindung zu Base (bevorzugt Alkalimetallhydroxid) liegt vorzugsweise im Bereich von 1 : 0,8 bis 1 : 5, besonders bevorzugt 1 : 1 bis 1 : 3,5. Falls in Schritt a) zusätzlich zu dem mit Schritt e) zurückgeführten wässrigen Extrakt noch eine wässrige Lösung einer Base zugegeben wird, erfolgt diese Zugabe vorzugsweise so, dass sich in der wässrigen Phase der Reaktionsmischung ein pH-Wert im Bereich von 8 bis 14, vorzugsweise 11 bis 14 ergibt. Die Umsetzung erfolgt vorzugsweise bei einer Temperatur im Bereich von -10 bis 50 °C, besonders bevorzugt 10 bis 40 °C.

Bei der Umsetzung eines Chlorformiats der Struktur R¹OC(O)Cl mit einem Hydroperoxid der Struktur R²OOH wird in Schritt a) ein Peroxomonocarbonat der Struktur R¹OC(O)OOR² erhalten. Das Chlorformiat und das Hydroperoxid werden dabei vorzugsweise in einem Molverhältnis von 1 : 1,01 bis 1 : 2, besonders bevorzugt 1 : 1,01 bis 1 : 1,5 eingesetzt. Das Molverhältnis von Chlorformiat zu Base (bevorzugt Alkalimetallhydroxid) liegt vorzugsweise im Bereich von 1 : 1 bis 1 : 2, besonders bevorzugt 1 : 1,2 bis 1 : 1,5. Falls in Schritt a) zusätzlich zu dem mit Schritt e) zurückgeführten wässrigen Extrakt noch eine wässrige Lösung einer Base zugegeben wird, erfolgt diese Zugabe vorzugsweise so, dass sich in der wässrigen Phase der Reaktionsmischung ein pH-Wert im Bereich von 8 bis 14, vorzugsweise 10 bis 14 ergibt. Die Umsetzung erfolgt vorzugsweise bei einer Temperatur im Bereich von -10 bis 50 °C, besonders bevorzugt 0 bis 40 °C.

Als Carbonsäurechlorid wird vorzugsweise eine Verbindung aus der Reihe Acetylchlorid, Propionylchlorid, Butyrylchlorid, Isobutyrylchlorid, Valeroylchlorid, 2-Methylbutyrylchlorid, Pivaloylchlorid, 2-Methylpentanoylchlorid, 2-Ethylbutyrylchlorid, 2-Ethylhexanoylchlorid, Nonanoylchlorid, 2,4,4-Trimethylpentanoylchlorid, 3,5,5-Trimethylhexanoylchlorid, Decanoylchlorid, Neodecanoylchlorid, Lauroylchlorid, Benzoylchlorid, 2-Methylbenzoylchlorid, 4-Methylbenzoylchlorid, 4-Chlorbenzoylchlorid, 2,4-Dichlorbenzoylchlorid und Naphthoylchlorid eingesetzt. Besonders bevorzugt wird als Carbonsäurechlorid Pivaloylchlorid, 2-Ethylhexanoylchlorid oder Benzoylchlorid verwendet.

Als Carbonsäureanhydrid wird vorzugsweise eine Verbindung aus der Reihe Acetanhydrid, Bernsteinsäureanhydrid, Maleinsäureanhydrid und Phthalsäureanhydrid eingesetzt.

Als Chlorformiat wird vorzugsweise eine Verbindung aus der Reihe Methylchlorformiat, Ethylchlorformiat, n-Propylchlorformiat, Isopropylchlorformiat, n-Butylchlorformiat, sec-Butylchlorformiat, 2-Ethylhexylchlorformiat, Isotridecylchlorformiat, Myristylchlorformiat, Cetylchlorformiat, Stearylchlorformiat, Cyclohexylchlorformiat, 4-tert-Butylcyclohexylchlorformiat, Benzylchlorformiat und 2-Phenoxyethylchlorformiat eingesetzt. Besonders bevorzugt wird als Chlorformiat 2-Ethylhexylchlorformiat verwendet.

Als organisches Hydroperoxid wird vorzugsweise eine Verbindung aus der Reihe tert-Butylhydroperoxid, tert-Amylhydroperoxid, 1,1,3,3-Tetramethylbutylhydroperoxid, 2,5-Dimethylhexan-2,5-dihydroperoxid, 2,5-Dimethyl-3-hexin-2,5-dihydroperoxid, p-Menthanhydroperoxid, Pinanhydroperoxid, Tetralinhydroperoxid, Cumolhydroperoxid, 4-tert-Butylcumolhydroperoxid, 1,3-Diisopropylbenzoldihydroperoxid und 1,4-Diisopropylbenzoldihydroperoxid eingesetzt. Besonders bevorzugt wird als organisches Hydroperoxid tert-Butylhydroperoxid verwendet.

In Schritt b) erfolgt die Einstellung des pH-Werts vorzugsweise in einem kontinuierlich betriebenen Rührkessel, in dem der pH-Wert der wässrigen Phase durch eine pH-geregelte Zugabe einer Säure oder Lauge auf dem gewünschten Wert gehalten wird.

Die Phasentrennung in Schritt c) erfolgt in bekannter Weise. Vorzugsweise werden die Schritte b) und c) in einer dem Fachmann als Mixer-Settler bekannten Vorrichtung kontinuierlich durchgeführt.

Die Extraktion in Schritt d) wird vorzugsweise mit einer wässrigen Lösung durchgeführt, die mehr als 0,5 mol/l und besonders bevorzugt mehr als 2 mol/l Base enthält. Vorzugsweise wird als Base ein Alkalimetallhydroxid und besonders bevorzugt Natriumhydroxid oder Kaliumhydroxid verwendet. Durch die Verwendung einer hohen Konzentration an Base kann die Menge an Wasser, die im Schritt e) in die Umsetzung von Schritt a) zurückgeführt wird, gering gehalten werden. Dies hat den Vorteil, dass die in den Schritten a) bis d) verwendeten Apparate ein kleineres Volumen aufweisen können.

Für die Extraktion in Schritt d) können alle dem Fachmann bekannten Apparate für flüssig-flüssig Extraktion verwendet werden. Vorzugsweise wird die Extraktion kontinuierlich durchgeführt, besonders bevorzugt in Form einer Gegenstromextraktion, bei der die organische Phase und die wässrige Lösung der Base im Gegenstrom durch den Extraktionsapparat geführt werden. Eine Gegenstromextraktion wird dabei vorzugsweise so ausgelegt, dass für die Extraktion des organischen Hydroperoxids in die wässrige Phase eine Trennwirkung von 2 bis 10 theoretischen Stufen erzielt wird.

Die Extraktion in Schritt d) wird vorzugsweise so durchgeführt, dass die Kontaktzeit zwischen der organischen Phase und der wässrigen Lösung weniger als 20 Minuten, besonders bevorzugt weniger als 2 Minuten, beträgt. Durch die Beschränkung der Kontaktzeit lässt sich weitgehend verhindern, dass Acylperoxid durch die wässrige Lösung der Base zu einem Carboxylatsalz und Hydroperoxid verseift wird. Der Produktverlust durch Verseifung lässt sich außerdem gering halten, indem die Extraktion vorzugsweise bei einer Temperatur im Bereich von 10 bis 50 °C, besonders bevorzugt 15 bis 30 °C durchgeführt wird.

In einer besonders bevorzugten Ausführungsform wird die Extraktion in Schritt d) in einem Zentrifugalextraktor durchgeführt, der insbesondere so ausgelegt ist, dass eine mehrstufige Gegenstromextraktion mit einer Kontaktzeit zwischen organischer Phase und wässriger Lösung von weniger als 2 Minuten erfolgt. Geeignete Zentrifugalextraktoren sind dem Fachmann aus dem Stand der Technik bekannt, beispielsweise aus Ullmann's Encyclopedia of Chemical Technology, Vol. B3, Seiten 6-21 bis 6-22.

Die aus der Extraktion in Schritt d) erhaltene organische Phase enthält das Acylperoxid und gegebenenfalls ein in Schritt a) zugesetztes Lösungsmittel. Diese organische Phase wird vorzugsweise noch mindestens einer Wäsche unterzogen, um Reste von in der organischen Phase dispergierter, basisch reagierender wässriger Phase und gegebenenfalls Reste von Hydroperoxid zu entfernen. Die Wäsche erfolgt vorzugsweise mit wässriger Säure, der zur Entfernung von Hydroperoxid ein geeignetes, vorzugsweise in der organischen Phase wenig lösliches Reduktionsmittel zugesetzt werden kann. Bei Verwendung eines Zentrifugalextraktors kann die Wäsche vorteilhaft in einem der Extraktion von Schritt d) nachgeschalteten Abschnitt des Apparats erfolgen, aus dem die gebrauchte Waschlösung getrennt vom wässrigen Extrakt von Schritt d) erhalten wird.

Aus der in Schritt d) erhaltenen organischen Phase wird, gegebenenfalls nach vorheriger Wäsche, vorzugsweise noch das in der organischen Phase gelöste und/oder dispergierte Wasser durch Trocknung entfernt. Die Trocknung kann dabei durch Wasser aufnehmende Absorbentien, durch Trocknung im Vakuum oder durch Strippen mit einem Gasstrom bewirkt werden. Vorzugsweise wird eine Trocknung durch Strippen mit einem Gasstrom bei einem Druck im Bereich von 20 bis 100 mbar und einer Temperatur im Bereich von 10 bis 50 °C durchgeführt.

Das erfindungsgemäße Verfahren ermöglicht es, die Herstellung von Acylperoxiden aus einer Acylverbindung und einem organischen Hydroperoxid unter Verwendung eines Überschusses an Hydroperoxid durchzuführen, ohne dass für die Umsetzung eine gegenüber der stöchimetrischen Menge erhöhte Menge an Hydroperoxid verbraucht wird. Durch die Verwendung eines Überschusses an Hydroperoxid kann die Umsetzung rascher zu hohen Umsätzen an Acylverbindung geführt werden, so dass in Schritt a) ein kleineres Reaktionsvolumen erforderlich ist, was neben Einsparungen bei den Apparaten auch zu einer höheren Sicherheit wegen der geringeren Menge an Acylperoxid im Reaktor führt. Da bei dem erfindungsgemäßen Verfahren mit den ausgeschleusten wässrigen Phasen geringere Mengen an gelöstem Hydroperoxid ausgetragen werden, ergibt sich mit dem erfindungsgemäßen Verfahren auch eine geringere Abwasserbelastung. Durch die Abtrennung von nicht umgesetztem Hydroperoxid wird mit dem erfindungsgemäßen Verfahren außerdem ein Produkt mit höherer Reinheit erhalten.

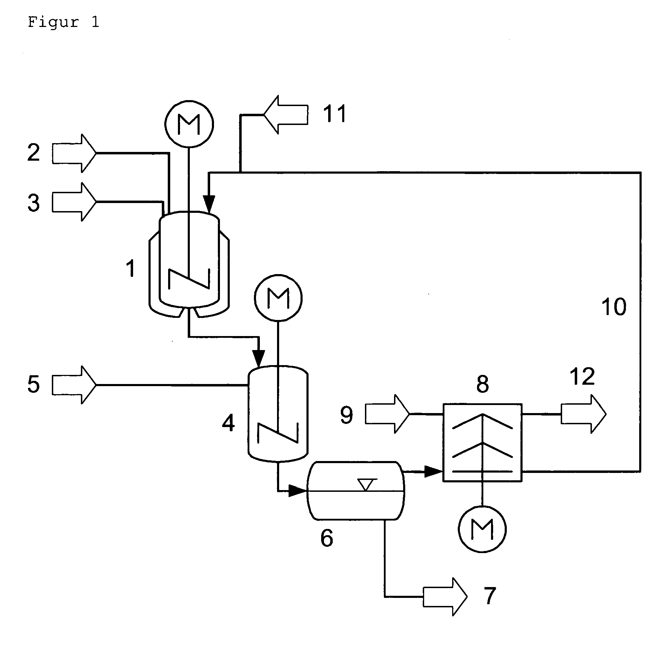

Figur 1 zeigt eine bevorzugte Ausführungsform des erfindungsgemäßen Verfahrens unter Verwendung eines Zentrifugalextraktors.

Bei der in Figur 1 gezeigten bevorzugten Ausführungsform wird Schritt a) in einem Rührkessel mit Kühlmantel (1) durchgeführt, dem eine Acylverbindung (2) und ein organisches Hydroperoxid (3) zugeführt wird. Schritt b) wird in einem Rührbehälter (4) durchgeführt, dem die zweiphasige Reaktionsmischung aus Schritt a) zugeführt wird. Im Rührbehälter (4) wird durch Zugabe von Säure (5) in der wässrigen Phase der gewünschte pH-Wert eingestellt. Die in Schritt b) erhaltene zweiphasige Mischung wird einem Behälter (6) zugeführt, in dem Schritt c) erfolgt, die Trennung in eine wässrige Phase und eine organische Phase. Die in Schritt c) erhaltene wässrige Phase (7) wird ausgeschleust. Die in Schritt c) erhaltene organische Phase wird einem Zentrifugalextraktor (8) zugeführt, in dem sie in einem Schritt d) mit einer wässrigen Lösung eines Alkalimetallhydroxids (9) extrahiert wird. Der in Schritt d) erhaltene wässrige Extrakt wird in einem Schritt e) über eine Leitung (10) in den Rührkessel (1) von Schritt a) zurückgeführt. Die in Schritt a) zusätzlich zu der in der Lösung (9) enthaltenen Menge an Alkalimetallhydroxid benötigte Menge an Alkalimetallhydroxid wird als wässrige Lösung von Alkalimetallhydroxid (11) dem Schritt a) zugeführt. Die in Schritt e) erhaltene organische Phase, die das Acylperoxid enthält und aus der nicht umgesetztes Hydroperoxid entfernt wurde, kann weiteren, nicht gezeigten Schritten zum Waschen und Trocknen unterworfen werden.

Die folgenden Beispiele illustrieren das erfindungsgemäße Verfahren.

### Beispiele

### Versuchsdurchführung

Die Umsetzung erfolgt in einer Reaktoranordnung aus einem Schlaufenreaktor mit 2,2 l Volumen, in dem die Reaktionsmischung mit einer Pumpe mit einer Flussrate von 1 m³/h durch einen Wärmeaustauscher geführt wird und einem nachgeschalteten Rührzellenreaktor mit 6 übereinander angeordneten Kammern von je 1,7 l Volumen und gemeinsamen Kühlmantel, wobei benachbarte Kammern jeweils über Durchlässe miteinander verbunden sind. In jeder Kammer ist ein Rührer angebracht, wobei alle Rührer über eine gemeinsame Welle angetrieben werden. Der Rührzellenreaktor entspricht damit 6 in Reihe geschalteten kontinuierlichen Rührkesselreaktoren. Die Einsatzstoffe werden in den Schlaufenreaktor in die Verbindungsleitung unmittelbar vor der Umwälzpumpe eingespeist. Die dem Schlaufenreaktor entnommene Reaktionsmischung wird der untersten Kammer des Rührzellenreaktors zugeführt, die Entnahme der umgesetzten Reaktionsmischung erfolgt durch Überlauf aus der obersten Kammer des Rührzellenreaktors. Die Reaktionsmischung wird dann einem kontinuierlichen, gekühlten Rührkessel zugeführt, in dem durch Zugabe von wässriger Salzsäure eine partielle Neutralisation auf einen pH-Wert im Bereich von 10 bis 12 erfolgt und gegebenenfalls Lösungsmittel zugesetzt wird. Die partiell neutralisierte Reaktionsmischung wird danach in einem Absetzbehälter in eine wässrige und eine organische Phase getrennt. Die organische Phase wird anschließend in einem Zentrifugalextraktor im Gegenstrom mit einer wässrigen Lösung von Kaliumhydroxid extrahiert und der erhaltene wässrige Extrakt wird in den Schlaufenreaktor zurückgeführt. Die organische Phase wird nach der Extraktion gewaschen und durch Strippen in einer Füllkörperkolonne im Vakuum getrocknet.

### Beispiel 1

### Herstellung von tert-Butylperoxypivalat

Der Schlaufenreaktor wird vor Reaktionsbeginn mit einer Lösung von 26,0 Gew.-% tert-Butylhydroperoxid und 16,2 Gew.-% Kaliumhydroxid in Wasser gefüllt. Dann werden dem Schlaufenreaktor zunächst 26,0 kg/h einer Lösung von 70 Gew.-% tert-Butylhydroperoxid in Wasser, 25,0 kg/h einer Lösung von 45 Gew.-% Kaliumhydroxid in Wasser, 26,4 kg/h Wasser und 18,0 kg/h Pivaloylchlorid zugeführt. Durch Kühlen mit Kühlwasser wird die Innentemperatur im Schlaufenreaktor auf 15 °C und im Rührzellenreaktor auf 10 °C gehalten. Die partielle Neutralisation erfolgt mit 6,0 kg/h 31 Gew.-% Salzsäure unter Zusatz von 8,2 kg/h Isododecan bei einer Temperatur von 8 °C. Die Extraktion der organischen Phase erfolgt mit 33,2 kg/h einer 20 Gew.-% wässrigen Lösung von Kaliumhydroxid. Dabei werden 37,4 kg/h wässriger Extrakt mit 11,6 Gew.-% tert-Butylhydroperoxid und 17,6 Gew.-% Kaliumhydroxid erhalten, die in den Schlaufenreaktor zurückgeführt werden. Ab dem Zeitpunkt, zu dem wässriger Extrakt in den Schlaufenreaktor zurückgeführt wird, wird die Dosierung der Einsatzstoffe auf 19,8 kg/h einer Lösung von 70 Gew.-% tert-Butylhydroperoxid in Wasser, 10,2 kg/h einer Lösung von 45 Gew.-% Kaliumhydroxid in Wasser und 9,8 kg/h Wasser geändert. Die organische Phase wird nach der Extraktion mit 36,0 kg/h einer Lösung von 1 Gew.-% Natriumsulfit und 0,2 Gew.-% Schwefelsäure gewaschen und durch Strippen bei 20 °C und 45 mbar getrocknet. Man erhält 32,4 kg/h einer 75,3 Gew.-% Lösung von tert-Butylperoxypivalat in Isododecan (93,7 % Ausbeute bezogen auf Pivaloylchlorid).

Im Betrieb mit Rückführung des extrahierten tert-Butylhydroperoxids liegt das Molverhältnis der Einsatzstoffe tert-Butylhydroperoxid zu Pivaloylchlorid bei 1,03 : 1.

### Beispiel 2

### Herstellung von tert-Butylperoxy-2-ethylhexanoat

Der Schlaufenreaktor wird vor Reaktionsbeginn mit einer Lösung von 25,8 Gew.-% tert-Butylhydroperoxid und 16,4 Gew.-% Kaliumhydroxid in Wasser gefüllt. Dann werden dem Schlaufenreaktor zunächst 24,6 kg/h einer Lösung von 70 Gew.-% tert-Butylhydroperoxid in Wasser, 24,0 kg/h einer Lösung von 45 Gew.-% Kaliumhydroxid in Wasser, 17,4 kg/h Wasser und 24,0 kg/h 2-Ethylhexanoylchlorid zugeführt. Durch Kühlen mit Kühlwasser wird die Innentemperatur im Schlaufenreaktor auf 35 °C und im Rührzellenreaktor auf 27 °C gehalten. Die partielle Neutralisation erfolgt mit 5,4 kg/h 31 Gew.-% Salzsäure unter Zusatz von 6,0 kg/h Wasser bei einer Temperatur von 18 °C. Die Extraktion der organischen Phase erfolgt mit 26,0 kg/h einer 15 Gew.-% wässrigen Lösung von Kaliumhydroxid. Dabei werden 29,2 kg/h wässriger Extrakt mit 10,8 Gew.-% tert-Butylhydroperoxid und 13,4 Gew.-% Kaliumhydroxid erhalten, die in den Schlaufenreaktor zurückgeführt werden. Ab dem Zeitpunkt, zu dem wässriger Extrakt in den Schlaufenreaktor zurückgeführt wird, wird die Dosierung der Einsatzstoffe auf 20,0 kg/h einer Lösung von 70 Gew.-% tert-Butylhydroperoxid in Wasser, 15,4 kg/h einer Lösung von 45 Gew.-% Kaliumhydroxid in Wasser und 1,4 kg/h Wasser geändert. Die organische Phase wird nach der Extraktion mit 36,0 kg/h einer Lösung von 1 Gew.-% Natriumsulfit und 0,2 Gew.-% Schwefelsäure gewaschen und durch Strippen bei 33 °C und 40 mbar getrocknet. Man erhält 31,6 kg/h tert-Butylperoxy-2-ethylhexanoat mit einer Reinheit von 99,3 % (98,5 % Ausbeute bezogen auf 2-Ethylhexanoylchlorid).

Im Betrieb mit Rückführung des extrahierten tert-Butylhydroperoxids liegt das Molverhältnis der Einsatzstoffe tert-Butylhydroperoxid zu 2-Ethylhexanoylchlorid bei 1,05 : 1.

### Beispiel 3

### Herstellung von tert-Amylperoxypivalat

Der Schlaufenreaktor wird vor Reaktionsbeginn mit einer Lösung von 23,1 Gew.-% tert-Amylhydroperoxid, 9,9 Gew.-% Kaliumhydroxid und 8,3 Gew.-% Natriumhydroxid in Wasser gefüllt. Dann werden dem Schlaufenreaktor zunächst 20,0 kg/h einer Lösung von 88 Gew.-% tert-Amylhydroperoxid in Wasser, 15,8 kg/h einer Lösung von 45 Gew.-% Kaliumhydroxid in Wasser, 11,8 kg/h einer Lösung von 50 Gew.-% Natriumhydroxid in Wasser, 24,2 kg/h Wasser und 18,0 kg/h Pivaloylchlorid zugeführt. Durch Kühlen mit Kühlwasser wird die Innentemperatur im Schlaufenreaktor auf 18 °C und im Rührzellenreaktor auf 10 °C gehalten. Die partielle Neutralisation erfolgt mit 11,4 kg/h 31 Gew.-% Salzsäure unter Zusatz von 7,4 kg/h Isododecan bei einer Temperatur von 10 °C. Die Extraktion der organischen Phase erfolgt mit 27,8 kg/h einer 16 Gew.-% wässrigen Lösung von Kaliumhydroxid. Dabei werden 29,4 kg/h wässriger Extrakt mit 5,8 Gew.-% tert-Amylhydroperoxid und 15,1 Gew.-% Kaliumhydroxid erhalten, die in den Schlaufenreaktor zurückgeführt werden. Ab dem Zeitpunkt, zu dem wässriger Extrakt in den Schlaufenreaktor zurückgeführt wird, wird die Dosierung der Einsatzstoffe auf 18,2 kg/h einer Lösung von 88 Gew.-% tert-Amylhydroperoxid in Wasser, 5,8 kg/h einer Lösung von 45 Gew.-% Kaliumhydroxid in Wasser und 6,6 kg/h Wasser geändert. Die organische Phase wird nach der Extraktion mit 36 kg/h einer Lösung von 5 Gew.-% Natriumsulfit und 0,2 Gew.-% Schwefelsäure gewaschen und durch Strippen bei 20 °C und 45 mbar getrocknet. Man erhält 34,4 kg/h einer 76,6 Gew.-% Lösung von tert-Amylperoxypivalat in Isododecan (93,9 % Ausbeute bezogen auf Pivaloylchlorid).

Im Betrieb mit Rückführung des extrahierten tert-Amylhydroperoxids liegt das Molverhältnis der Einsatzstoffe tert-Amylhydroperoxid zu Pivaloylchlorid bei 1,03 : 1.

### Beispiel 4

Herstellung von tert-Butylperoxy-3,5,5-trimethylhexanoat Der Schlaufenreaktor wird vor Reaktionsbeginn mit einer Lösung von 25,7 Gew.-% tert-Butylhydroperoxid und 15,0 Gew.-% Kaliumhydroxid in Wasser gefüllt. Dann werden dem Schlaufenreaktor zunächst 20,2 kg/h einer Lösung von 70 Gew.-% tert-Butylhydroperoxid in Wasser, 18,2 kg/h einer Lösung von 45 Gew.-% Kaliumhydroxid in Wasser, 16,2 kg/h Wasser und 19,2 kg/h 3,5,5-Trimethylhexanoylchlorid zugeführt. Durch Kühlen mit Kühlwasser wird die Innentemperatur im Schlaufenreaktor und im Rührzellenreaktor auf 25 °C gehalten. Die partielle Neutralisation erfolgt mit 27,6 kg/h 4 Gew.-% Salzsäure bei einer Temperatur von 15 °C. Die Extraktion der organischen Phase erfolgt mit 23,6 kg/h einer 20 Gew.-% wässrigen Lösung von Kaliumhydroxid. Dabei werden 26,2 kg/h wässriger Extrakt mit 9,6 Gew.-% tert-Butylhydroperoxid und 18,0 Gew.-% Kaliumhydroxid erhalten, die in den Schlaufenreaktor zurückgeführt werden. Ab dem Zeitpunkt, zu dem wässriger Extrakt in den Schlaufenreaktor zurückgeführt wird, wird die Dosierung der Einsatzstoffe auf 16,6 kg/h einer Lösung von 70 Gew.-% tert-Butylhydroperoxid in Wasser, 7,8 kg/h einer Lösung von 45 Gew.-% Kaliumhydroxid in Wasser und 4,8 kg/h Wasser geändert. Die organische Phase wird nach der Extraktion mit 33,8 kg/h einer Lösung von 3 Gew.-% Natriumsulfit und 1 Gew.-% Schwefelsäure gewaschen und durch Strippen bei 34 °C und 47 mbar getrocknet. Man erhält 24,6 kg/h tert-Butylperoxy-3,5,5-trimethylhexanoat mit einer Reinheit von 99,9 % (98,2 % Ausbeute bezogen auf 3,5,5-Trimethylhexanoylchlorid).

Im Betrieb mit Rückführung des extrahierten tert-Butylhydroperoxids liegt das Molverhältnis der Einsatzstoffe tert-Butylhydroperoxid zu 3,5,5-Trimethylhexanoylchlorid bei 1,19 : 1.

### Beispiel 5

### Herstellung von tert-Butylperoxyisobutyrat

Der Schlaufenreaktor wird vor Reaktionsbeginn mit einer Lösung von 24,4 Gew.-% tert-Butylhydroperoxid und 12,7 Gew.-% Kaliumhydroxid in Wasser gefüllt. Dann werden dem Schlaufenreaktor zunächst 33,6 kg/h einer Lösung von 70 Gew.-% tert-Butylhydroperoxid in Wasser, 27,0 kg/h einer Lösung von 45 Gew.-% Kaliumhydroxid in Wasser, 35,0 kg/h Wasser und 18,4 kg/h Isobutyroylchlorid zugeführt. Durch Kühlen mit Kühlwasser wird die Innentemperatur im Schlaufenreaktor auf 11 °C und im Rührzellenreaktor auf 12 °C gehalten. Die partielle Neutralisation erfolgt mit 3,6 kg/h 31 Gew.-% Salzsäure unter Zusatz von 8,0 kg/h Isododecan bei einer Temperatur von 8 °C. Die Extraktion der organischen Phase erfolgt mit 33,0 kg/h einer 15 Gew.-% wässrigen Lösung von Kaliumhydroxid. Dabei werden 39,8 kg/h wässriger Extrakt mit 17,1 Gew.-% tert-Butylhydroperoxid und 12,2 Gew.-% Kaliumhydroxid erhalten, die in den Schlaufenreaktor zurückgeführt werden. Ab dem Zeitpunkt, zu dem wässriger Extrakt in den Schlaufenreaktor zurückgeführt wird, wird die Dosierung der Einsatzstoffe auf 23,8 kg/h einer Lösung von 70 Gew.-% tert-Butylhydroperoxid in Wasser, 16,0 kg/h einer Lösung von 45 Gew.-% Kaliumhydroxid in Wasser und 15,8 kg/h Wasser geändert. Die organische Phase wird nach der Extraktion mit 36 kg/h einer Lösung von 3 Gew.-% Natriumsulfit und 1 Gew.-% Schwefelsäure gewaschen und durch Strippen bei 18 °C und 58 mbar getrocknet. Man erhält 33,4 kg/h einer 76,5 Gew.-% Lösung von tert-Butylperoxyisobutyrat in Isododecan (92,8 % Ausbeute bezogen auf Isobutyroylchlorid).

Im Betrieb mit Rückführung des extrahierten tert-Butylhydroperoxids liegt das Molverhältnis der Einsatzstoffe tert-Butylhydroperoxid zu Isobutyroylchlorid bei 1,07 : 1.

### Beispiel 6

### Herstellung von tert-Butylperoxybenzoat

Der Schlaufenreaktor wird vor Reaktionsbeginn mit einer Lösung von 19,6 Gew.-% tert-Butylhydroperoxid und 9,0 Gew.-% Natriumhydroxid in Wasser gefüllt. Dann werden dem Schlaufenreaktor zunächst 18,4 kg/h einer Lösung von 70 Gew.-% tert-Butylhydroperoxid in Wasser, 11,8 kg/h einer Lösung von 50 Gew.-% Natriumhydroxid in Wasser, 35,2 kg/h Wasser und 18,0 kg/h Benzoylchlorid zugeführt. Durch Kühlen mit Kühlwasser wird die Innentemperatur im Schlaufenreaktor auf 12 °C und im Rührzellenreaktor auf 11 °C gehalten. Die partielle Neutralisation erfolgt mit 2,2 kg/h 31 Gew.-% Salzsäure unter Zusatz von 6,0 kg/h Wasser bei einer Temperatur von 16 °C. Die Extraktion der organischen Phase erfolgt mit 17,2 kg/h einer 15 Gew.-% wässrigen Lösung von Natriumhydroxid. Dabei werden 18,4 kg/h wässriger Extrakt mit 6,3 Gew.-% tert-Butylhydroperoxid und 14,1 Gew.-% Natriumhydroxid erhalten, die in den Schlaufenreaktor zurückgeführt werden. Ab dem Zeitpunkt, zu dem wässriger Extrakt in den Schlaufenreaktor zurückgeführt wird, wird die Dosierung der Einsatzstoffe auf 16,8 kg/h einer Lösung von 70 Gew.-% tert-Butylhydroperoxid in Wasser, 6,6 kg/h einer Lösung von 50 Gew.-% Natriumhydroxid in Wasser und 23,0 kg/h Wasser geändert. Die organische Phase wird nach der Extraktion mit 36 kg/h einer Lösung von 1 Gew.-% Natriumsulfit und 0,4 Gew.-% Schwefelsäure gewaschen und durch Strippen bei 35 °C und 43 mbar getrocknet. Man erhält 23,8 kg/h tert-Butylperoxybenzoat mit einer Reinheit von 99,7 % (95,3 % Ausbeute bezogen auf Benzoylchlorid).

Im Betrieb mit Rückführung des extrahierten tert-Butylhydroperoxids liegt das Molverhältnis der Einsatzstoffe tert-Butylhydroperoxid zu Benzoylchlorid bei 1,02 : 1.

## Patentansprüche

1. Verfahren zur Herstellung von Acylperoxiden durch Umsetzung einer Acylverbindung aus der Reihe der Carbonsäurechloride, Carbonsäureanhydride und Chlorformiate mit einem organischen Hydroperoxid, umfassend die Schritte
a) Umsetzung der Acylverbindung, des organischen Hydroperoxids und einer wässrigen Lösung einer Base unter Erhalt einer zweiphasigen Reaktionsmischung,
b) Einstellung des pH-Werts der wässrigen Phase der in Schritt a) erhaltenen Reaktionsmischung auf einen Wert im Bereich von 6 bis 13,
c) Trennung der in Schritt b) erhaltenen zweiphasigen Mischung in eine wässrige und eine organische Phase,
d) Extraktion der in Schritt c) erhaltenen organischen Phase mit einer wässrigen Lösung einer Base und
e) Rückführung des in Schritt d) erhaltenen wässrigen Extrakts in Schritt a).

2. Verfahren nach Anspruch 1,
**dadurch gekennzeichnet,**
**dass** in Schritt a) das erhaltene Reaktionsgemisch auf einen pH-Wert im Bereich von 11 bis 12,5 eingestellt wird.

3. Verfahren nach Anspruch 1,
**dadurch gekennzeichnet,**
**dass** in Schritt a) das Molverhältnis von organischem Hydroperoxid zu Acylverbindung im Bereich von 1,01 bis 2 liegt.

4. Verfahren nach einem der Ansprüche 1 bis 3,
**dadurch gekennzeichnet,**
**dass** in Schritt a) das Molverhältnis von organischem Hydroperoxid zu Acylverbindung im Bereich von 1,05 bis 1,5 liegt.

5. Verfahren nach einem der Ansprüche 1 bis 4,
**dadurch gekennzeichnet,**
**dass** in Schritt d) die wässrige Lösung einer Base einen Gehalt von mehr als 0,5 mol/l Base aufweist.

6. Verfahren nach einem der Ansprüche 1 bis 5,
**dadurch gekennzeichnet,**
**dass** in Schritt d) die wässrige Lösung einer Base einen Gehalt von mehr als 2 mol/l aufweist.

7. Verfahren nach einem der vorhergehenden Ansprüche,
**dadurch gekennzeichnet,**
**dass** die Extraktion in Schritt d) als Gegenstromextraktion durchgeführt wird.

8. Verfahren nach einem der vorhergehenden Ansprüche,
**dadurch gekennzeichnet,**
**dass** in Schritt d) die Kontaktzeit zwischen der organischen Phase und der wässrigen Lösung weniger als 20 Minuten beträgt.

9. Verfahren nach einem der vorhergehenden Ansprüche,
**dadurch gekennzeichnet,**
**dass** in Schritt d) die Kontaktzeit zwischen der organischen Phase und der wässrigen Lösung weniger als 2 Minuten beträgt.

10. Verfahren nach einem der vorhergehenden Ansprüche,
**dadurch gekennzeichnet,**
**dass** die Extraktion in einem Zentrifugalextraktor durchgeführt wird.

11. Verfahren nach einem der vorhergehenden Ansprüche,
**dadurch gekennzeichnet,**
**dass** die Acylverbindung ein Carbonsäurechlorid aus der Reihe Acetylchlorid, Propionylchlorid, Butyrylchlorid, Isobutyrylchlorid, Valeroylchlorid, 2-Methylbutyrylchlorid, Pivaloylchlorid, 2-Methylpentanoylchlorid, 2-Ethylbutyrylchlorid, 2-Ethylhexanoylchlorid, Nonanoylchlorid, 2,4,4-Trimethylpentanoylchlorid, 3,5,5-Trimethylhexanoylchlorid, Decanoylchlorid, Neodecanoylchlorid, Lauroylchlorid, Benzoylchlorid, 2-Methylbenzoylchlorid, 4-Methylbenzoylchlorid, 4-Chlorbenzoylchlorid, 2,4-Dichlorbenzoylchlorid und Naphthoylchlorid ist.

12. Verfahren nach einem der vorhergehenden Ansprüche,
**dadurch gekennzeichnet,**
**dass** die Acylverbindung ein Chlorformiat aus der Reihe Methylchlorformiat, Ethylchlorformiat, n-Propylchlorformiat, Isopropylchlorformiat, n-Butylchlorformiat, sec-Butylchlorformiat, 2-Ethylhexylchlorformiat, Isotridecylchlorformiat, Myristylchlorformiat, Cetylchlorformiat, Stearylchlorformiat, Cyclohexylchlorformiat, 4-tert-Butylcyclohexylchlorformiat, Benzylchlorformiat und 2-Phenoxyethylchlorformiat ist.

13. Verfahren nach einem der vorhergehenden Ansprüche,
**dadurch gekennzeichnet,**
**dass** das organische Peroxid aus der Reihe tert-Butylhydroperoxid, tert-Amylhydroperoxid, 1,1,3,3-Tetramethylbutylhydroperoxid, 2,5-Dimethylhexan-2,5-dihydroperoxid, 2,5-Dimethyl-3-hexin-2,5-dihydroperoxid, p-Menthanhydroperoxid, Pinanhydroperoxid, Tetralinhydroperoxid, Cumolhydroperoxid, 4-tert-Butylcumolhydroperoxid, 1,3-Diisopropylbenzoldihydroperoxid und 1,4-Diisopropylbenzoldihydroperoxid ausgewählt ist.

## Claims

1. Process for preparing acyl peroxides by reacting an acyl compound from the group of the acid chlorides, carboxylic anhydrides and chloroformates with an organic hydroperoxide, comprising the steps of
a) reacting the acyl compound, the organic hydroperoxide and an aqueous solution of a base to obtain a biphasic reaction mixture,
b) adjusting the pH of the aqueous phase of the reaction mixture obtained in step a) to a value in the range of 6 to 13,
c) separating the biphasic mixture obtained in step b) into an aqueous phase and an organic phase,
d) extracting the organic phase obtained in step c) with an aqueous solution of a base and
e) recycling the aqueous extract obtained in step d) into step a).

2. Process according to Claim 1,
**characterised in that**
the reaction mixture obtained in step a) is adjusted to a pH value in the range of 11 to 12.5.

3. Process according to Claim 1,
**characterised in that**
the molar ratio of organic hydroperoxide to acyl compound in step a) is in the range of 1.01 to 2.

4. Process according to one of Claims 1 to 3,
**characterised in that**
the molar ratio of organic hydroperoxide to acyl compound in step a) is in the range of 1.05 to 1.5.

5. Process according to one of Claims 1 to 4,
**characterised in that**
the aqueous solution of a base in step d) has a content of more than 0.5 mol/l of base.

6. Process according to one of Claims 1 to 5,
**characterised in that**
the aqueous solution of a base in step d) has a content of more than 2 mol/l of base.

7. Process according to one of the preceding claims,
**characterised in that**
the extraction in step d) is performed as a countercurrent extraction.

8. Process according to one of the preceding claims,
**characterised in that**
the contact time between the organic phase and the aqueous solution in step d) is less than 20 minutes.

9. Process according to one of the preceding claims,
**characterised in that**
the contact time between the organic phase and the aqueous solution in step d) is less than 2 minutes.

10. Process according to one of the preceding claims,
**characterised in that**
the extraction is performed in a centrifugal extractor.

11. Process according to one of the preceding claims,
**characterised in that**
the acyl compound is an acid chloride from the group of acetyl chloride, propionyl chloride, butyryl chloride, isobutyryl chloride, valeroyl chloride, 2-methylbutyryl chloride, pivaloyl chloride, 2-methylpentanoyl chloride, 2-ethylbutyryl chloride, 2-ethylhexanoyl chloride, nonanoyl chloride, 2,4,4-trimethylpentanoyl chloride, 3,5,5-trimethylhexanoyl chloride, decanoyl chloride, neodecanoyl chloride, lauroyl chloride, benzoyl chloride, 2-methylbenzoyl chloride, 4-methylbenzoyl chloride, 4-chlorobenzoyl chloride, 2,4-dichlorobenzoyl chloride and naphthoyl chloride.

12. Process according to one of the preceding claims,
**characterised in that**
the acyl compound is a chloroformate from the group of methyl chloroformate, ethyl chloroformate, n-propyl chloroformate, isopropyl chloroformate, n-butyl chloroformate, sec.-butyl chloroformate, 2-ethylhexyl chloroformate, isotridecyl chloroformate, myristyl chloroformate, cetyl chloroformate, stearyl chloroformate, cyclohexyl chloroformate, 4-tert.-butylcyclohexyl chloroformate, benzyl chloroformate and 2-phenoxyethyl chloroformate.

13. Process according to one of the preceding claims,
**characterised in that**
the organic peroxide is selected from the group of tert.-butyl hydroperoxide, tert.-amyl hydroperoxide, 1,1,3,3-tetramethylbutyl hydroperoxide, 2,5-dimethylhexane 2,5-dihydroperoxide, 2,5-dimethyl-3-hexyne 2,5-dihydroperoxide, p-menthane hydroperoxide, pinane hydroperoxide, tetralin hydroperoxide, cumene hydroperoxide, 4-tert.-butylcumene hydroperoxide, 1,3-diisopropylbenzene dihydroperoxide and 1,4-diisopropylbenzene dihydroperoxide.

## Revendications

1. Procédé de production de peroxydes d'acyle par réaction d'un composé acyle choisi dans la série des chlorures d'acide carboxylique, des anhydrides d'acide carboxylique et des chloroformiates avec un hydroperoxyde organique, comprenant les étapes consistant à :
a) faire réagir le composé acyle, l'hydroperoxyde organique et une solution aqueuse d'une base de manière à obtenir un mélange réactionnel biphasique,
b) ajuster le pH de la phase aqueuse du mélange réactionnel obtenu à l'étape a) à une valeur située dans la plage de 6 à 13,
c) séparer le mélange biphasique obtenu à l'étape b) en une phase aqueuse et une phase organique,
d) extraire la phase organique obtenue à l'étape c) avec une solution aqueuse d'une base et
e) recycler l'extrait aqueux obtenu à l'étape d) vers l'étape a).

2. Procédé selon la revendication 1, **caractérisé en ce que**, à l'étape a), le mélange réactionnel obtenu est ajusté à un pH situé dans la plage de 11 à 12,5.

3. Procédé selon la revendication 1, **caractérisé en ce que**, à l'étape a), le rapport molaire de l'hydroperoxyde organique au composé acyle est situé dans la plage de 1,01 à 2.

4. Procédé selon l'une des revendications 1 à 3, **caractérisé en ce que**, à l'étape a), le rapport molaire de l'hydroperoxyde organique au composé acyle est situé dans la plage de 1,05 à 1,5.

5. Procédé selon l'une des revendications 1 à 4, **caractérisé en ce que**, à l'étape d), la solution aqueuse d'une base présente une teneur en base supérieure à 0,5 mole/litre de base.

6. Procédé selon l'une des revendications 1 à 5, **caractérisé en ce que**, à l'étape d), la solution aqueuse d'une base présente une teneur en base supérieure à 2 moles/litre.

7. Procédé selon l'une des revendications précédentes, **caractérisé en ce que** l'extraction à l'étape d) est réalisée sous la forme d'une extraction à contre-courant.

8. Procédé selon l'une des revendications précédentes, **caractérisé en ce que**, à l'étape d), le temps de contact entre la phase organique et la solution aqueuse est inférieur à 20 minutes.

9. Procédé selon l'une des revendications précédentes, **caractérisé en ce que**, à l'étape d), le temps de contact entre la phase organique et la solution aqueuse est inférieur à 2 minutes.

10. Procédé selon l'une des revendications précédentes, **caractérisé en ce que** l'extraction s'effectue dans un extracteur centrifuge.

11. Procédé selon l'une des revendications précédentes, **caractérisé en ce que** le composé acyle est un chlorure d'acide carboxylique choisi dans le groupe formé par chlorure d'acétyle, chlorure de propionyle, chlorure de butyryle, chlorure d'isobutyryle, chlorure de valéroyle, chlorure de 2-méthylbutyryle, chlorure de pivaloyle, chlorure de 2-méthylpentanoyle, chlorure de 2-éthylbutyryle, chlorure de 2-éthylhexanoyle, chlorure de nonanoyle, chlorure de 2,4,4-triméthylpentanoyle, chlorure de 3,5,5-triméthylhexanoyle, chlorure de décanoyle, chlorure de néodécanoyle, chlorure de lauroyle, chlorure de benzoyle, chlorure de 2-méthylbenzoyle, chlorure de 4-méthylbenzoyle, chlorure de 4-chlorobenzoyle, chlorure de 2,4-dichlorobenzoyle et chlorure de naphtoyle.

12. Procédé selon l'une des revendications précédentes, **caractérisé en ce que** le composé acyle est un chloroformiate choisi dans le groupe formé par chloroformiate de méthyle, chloroformiate d'éthyle, chloroformiate de n-propyle, chloroformiate d'isopropyle, chloroformiate de n-butyle, chloroformiate de secbutyle, chloroformiate de 2-éthylhexyle, chloroformiate d'isotridécyle, chloroformiate de myristyle, chloroformiate de cétyle, chloroformiate de stéaryle, chloroformiate de cyclohexyle, chloroformiate de 4-tert.-butylcyclohexyle, chloroformiate de benzyle et chloroformiate de 2-phénoxyéthyle.

13. Procédé selon l'une des revendications précédentes, **caractérisé en ce que** le peroxyde organique est choisi dans le groupe formé par hydroperoxyde de tert.-butyle, hydroperoxyde de tert.-amyle, hydroperoxyde de 1,1,3,3-tétraméthylbutyle, 2,5-dihydroperoxyde de 2,5-diméthylhexane, 2,5-dihydroperoxyde de 2,5-diméthyl-3-hexine, hydroperoxyde de p-menthane, hydroperoxyde de pinane, hydroperoxyde de tétraline, hydroperoxyde de cumène, hydroperoxyde de 4-tert.-butylcumène, dihydroperoxyde de 1,3-diisopropylbenzène et dihydroperoxyde de 1,4-diisopropylbenzène.
